(19) Europäisches Patentamt
European Patent Office
Office européen des brevets

(11) Veröffentlichungsnummer : **0 165 903**
**B1**

(12) # EUROPÄISCHE PATENTSCHRIFT

(45) Veröffentlichungstag der Patentschrift : **05.04.89**

(51) Int. Cl.[4] : **C 07 D309/14**, C 07 D405/12, A 01 N 47/34

(21) Anmeldenummer : **85810278.3**

(22) Anmeldetag : **17.06.85**

(54) **Phenylbenzoylharnstoffe.**

(30) Priorität : **22.06.84 CH 3030/84**
**31.05.85 CH 2316/85**

(43) Veröffentlichungstag der Anmeldung : **27.12.85 Patentblatt 85/52**

(45) Bekanntmachung des Hinweises auf die Patenterteilung : **05.04.89 Patentblatt 89/14**

(84) Benannte Vertragsstaaten : **AT BE CH DE FR IT LI NL SE**

(56) Entgegenhaltungen :
**FR-A- 2 029 220**
**US-A- 4 110 469**

(73) Patentinhaber : **CIBA-GEIGY AG**
**Klybeckstrasse 141**
**CH-4002 Basel (CH)**

(72) Erfinder : **Kristiansen, Odd, Dr.**
**Delligrabenstrasse 7**
**CH-4313 Möhlin (CH)**
Erfinder : **Drabek, Jozef, Dr.**
**Benkenstrasse 12**
**CH-4104 Oberwil (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Die vorliegende Erfindung betrifft neue N-Phenyl-N-tetrahydropyranyl-N'-benzoylharnstoffe, Verfahren zu ihrer Herstellung und ihre Verwendung in der Schädlingsbekämpfung.

Die erfindungsgemässen N-Phenyl-N-tetrahydropyranyl-N'-benzoylharnstoffe haben die Formel I

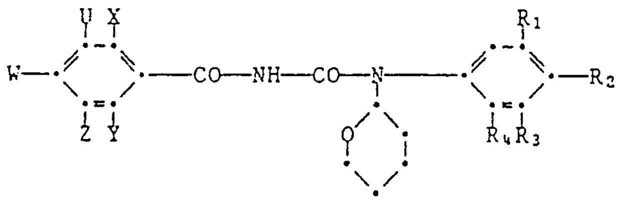

(I)

worin

$R_1$ Wasserstoff, Halogen, $C_1$-$C_6$-Alkyl, $C_1$-$C_3$-Halogenalkyl mit 1 bis 7 Halogenatomen, $C_1$-$C_6$-Alkoxy, $C_1$-$C_6$-Alkylthio, $C_1$-$C_3$-Halogenalkoxy mit 1 bis 7 Halogenatomen, Nitro, Cyano, Aethinyl oder eine der Gruppen

$-CO-OR_5$ oder 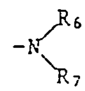

wobei $R_5$, $R_6$ und $R_7$ für $C_1$-$C_6$-Alkyl steht ;

$R_2$, $R_3$ und $R_4$ unabhängig voneinander Wasserstoff, Halogen, $C_1$-$C_6$-Alkyl, $C_1$-$C_3$-Halogenalkyl mit 1 bis 7 Halogenatomen, $C_1$-$C_6$-Alkoxy, $C_1$-$C_6$-Alkylthio, $C_1$-$C_3$-Halogenalkoxy mit 1 bis 7 Halogenatomen, Nitro, Cyano, Aethinyl oder eine der Gruppen

$-CO-OR_5$ oder 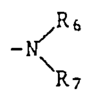

wobei $R_5$, $R_6$ und $R_7$ für $C_1$-$C_6$-Alkyl steht,

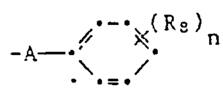

wobei —A— für —O— oder —S—, $R_8$ für Halogen, Methyl, Nitro, $CF_3$, oder Cyano und n für eine Zahl 0, 1, 2 oder 3 steht, oder

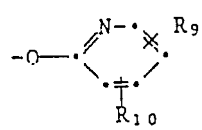

wobei $R_9$ und $R_{10}$ unabhängig voneinander für Wasserstoff, Chlor, Trifluormethyl oder einen mit Fluor- und/oder Chlor perhalogenierten Aethylrest stehen ; und

U, X, Y, Z und W unabhängig voneinander Wasserstoff, Halogen, $C_1$-$C_3$-Alkyl, $C_1$-$C_3$-Alkoxy oder $C_1$-$C_3$-Alkylthio

bedeuten.

Bevorzugt sind solche erfindungsgemässe Verbindungen der Formel I,

worin

$R_1$ Wasserstoff, Fluor, Chlor, Trifluormethyl, $—CO—OCH_3$ oder $—CO—OC_2H_5$ ;

$R_2$ Wasserstoff, Fluor, Chlor, $C_1$-$C_2$-Halogenalkyl mit 1 bis 5 Chlor- und/oder Fluoratomen, $C_1$-$C_2$-Halogenalkoxy mit 1 bis 5 Chlor- und/oder Fluoratomen, Cyano, Aethinyl, oder eine der Gruppen

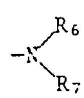

wobei $R_6$ und $R_7$ für $C_1$-$C_3$-Alkyl steht,

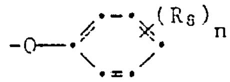

wobei $R_8$ für Fluor oder Chlor und n für eine Zahl 0, 1 oder 2 steht,

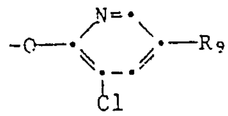

wobei $R_9$ für Wasserstoff, Chlor, Trifluormethyl oder $—CF_2—CFCl_2$ steht,

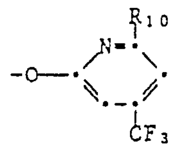

wobei $R_{10}$ für Wasserstoff oder Chlor steht ;
$R_3$ und $R_4$ unabhängig voneinander Wasserstoff, Halogen, Trifluormethyl, Methyl oder Aethyl ;
U, Z und W Wasserstoff ;
X Fluor, Chlor, Brom oder $C_1$-$C_3$-Alkyl ; und
Y Wasserstoff, Fluor, Chlor, Brom oder $C_1$-$C_3$-Alkyl
bedeuten,

Hervorzuheben sind insbesondere solche Verbindungen der Formel I, worin
$R_1$ Wasserstoff, Fluor, Chlor oder $—CO—OCH_3$ ;
$R_2$ Wasserstoff, Fluor, Chlor, Trifluormethyl, Aethinyl, Trifluormethoxy, $—O—CF_2—CF_2Cl$, $—O—CF_2—CHF_2$, $—O—CF_2—CFCl_2$ oder eine der Gruppen

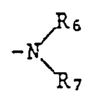

wobei $R_6$ für Methyl und $R_7$ für $C_1$-$C_3$-Alkyl steht,

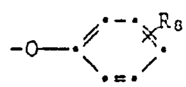

wobei $R_8$ für Wasserstoff, Fluor oder Chlor steht,

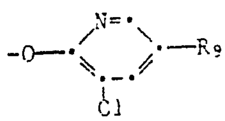

wobei $R_9$ für Trifluormethyl oder $—CF_2—CFCl_2$ steht, oder

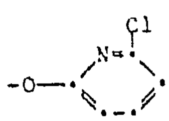

$R_3$ und $R_4$ unabhängig voneinander Wasserstoff, Fluor, Chlor, Trifluormethyl oder Methyl ;
U, Z und W Wasserstoff ;
X Fluor oder Chlor ; und
Y Wasserstoff, Fluor oder Chlor
bedeuten, sowie solche Verbindungen der Formel I, worin
$R_1$ Chlor oder Trifluormethyl ;
$R_2$ Chlor, Trifluormethyl, Trifluormethoxy, $—O—CF_2—CHF_2$ oder die Gruppe

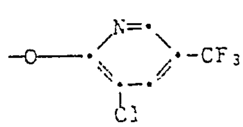

$R_3$ Wasserstoff, Chlor oder —CO—$OCH_3$ ;
$R_4$ Wasserstoff ;
U, Z und W Wasserstoff ;
X Fluor oder Chlor ; und
Y Wasserstoff oder Fluor
bedeuten.

Unter dem Begriff Alkyl sind im Rahmen der vorliegenden Erfindung geradkettige und verzweigte Alkylreste und je nach Zahl der angegebenen Kohlenstoffatome beispielsweise folgende Gruppen zu verstehen :

Methyl, Aethyl, Propyl, Butyl und Pentyl, sowie ihre Isomeren, wie z. B. Isopropyl, Isobutyl, tert.-Butyl, Isopentyl usw. Unter Halogen sind Fluor, Chlor, Brom und Jod, vorzugsweise Fluor und Chlor, zu verstehen.

Die Verbindungen der Formel I können analog an sich bekannter Verfahren hergestellt werden. So kann man z. B. eine Verbindung der Formel I erhalten, indem man eine Verbindung der Formel II

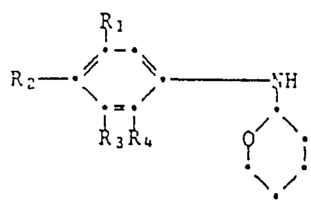

(II)

mit einer Verbindung der Formel III

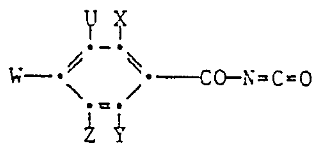

(III)

umsetzt, wobei in den Formeln II und III die Reste $R_1$, $R_2$, $R_3$, $R_4$, U, X, Y, Z und W die oben angegebenen Bedeutungen haben.

Das vorstehend erwähnte Verfahren kann vorzugsweise unter normalem Druck und in Gegenwart eines organischen Lösungs- oder Verdünnungsmittels durchgeführt werden. Als Lösungs- oder Verdünnungsmittel eignen sich z. B. Aether und ätherartige Verbindungen, wie Diäthyläther, Dipropyläther, Dibutyläther, Dioxan, Dimethoxyäthan und Tetrahydrofuran ; N,N-alkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid, sowie Ketone, z. B. Aceton, Methyläthylketon, Methylisopropylketon und Methylisobutylketon. Das Verfahren wird im allgemeinen bei einer Temperatur von —10 bis 100 °C, vorzugsweise zwischen 15 und 25 °C, gegebenenfalls in Gegenwart einer organischen Base, z. B. Triäthylamin, durchgeführt. Die Durchführung des Verfahrens erfolgt insbesondere bei Raumtemperatur.

Die als Ausgangsprodukte bei dem obigen Verfahren verwendeten N-Tetrahydropyranylaniline der Formel II können nach dem in J. org. chem. UdSSR 2034 (1976) beschriebenen Verfahren durch Umsetzung entsprechender Aniline der Formel IV

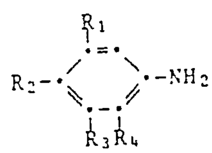

(IV)

mit 5-Hydroxypentanal hergestellt werden. Die erwähnten Aniline der Formel IV sind bekannt (vgl. z. B. deutsche Offenlegungsschriften Nr. 3241138A1 und 3240975A1).

Es ist bereits bekannt, dass N-Phenyl-N'-benzoylharnstoffe, die am Phenyl- sowie am Benzoylteil Substituenten unterschiedlicher Art aufweisen, insektizide Eigenschaften besitzen (vgl. z. B. die britische Patentschrift Nr. 1 324 293). Bei den Verbindungen gemäss vorliegender Erfindung handelt es sich demgegenüber um neuartige N-Phenyl-N-(tetrahydropyranyl-2'')-N'-benzoylharnstoffe, die strukturspezifisch in erster Linie durch die an das N-Atom gebundene Tetrahydropyranylgruppe charakterisiert sind. Diese neuen Verbindungen besitzen überraschenderweise ausgezeichnete Wirksamkeit als Schädlingsbekämpfungsmittel, insbesondere im Pflanzenschutz. Ein spezieller Vorteil der erfindungsgemässen Verbindungen der Formel I ergibt sich aus ihrer sehr geringen Warmblütertoxizität bei guter Pflanzenverträglichkeit.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z. B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z. B. Spodoptera littoralis und Heliothis virescens), sowie in Obst- und Gemüsekulturen (z. B. Laspeyresia pomonella, Leptinotarsa decemlineata und Epilachna varivestis) eingesetzt werden. Die Verbindungen der Formel I zeichnen sich durch ovizide und vor allem larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I können ferner zur Bekämpfung von Ektoparasiten, z. B. Zecken und Fliegen, wie Lucilia sericata, an Haus- und Nutztieren eingesetzt werden, und zwar bevorzugt durch Tier-, Stall- und Weidebehandlung. Ausgeschlossen soll dabei die Bekämpfung von Parasiten im Sinne einer am menschlichen oder tierischen Körper vorgenommenen therapeutischen Massnahme sein (gemäss Art. 52(4) EPÜ).

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Bryobia rubrioculus, Panonychus citri, Eriophyes piri, Eriophyes ribis, Eriophyes vitis, Tarsonemus pallidus, Phyllocoptes vitis und Phyllocoptruta oleivora.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z. B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d. h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Aret des zu formulierenden Wirkstoffes der Formel I oder der Kombinationen dieser Wirkstoffe mit anderen Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die die Alkali-, Erdalkali- oder gegebenenfalls substituierten Ammoniumsalze von höheren Fettsäuren ($C_{10}$-$C_{22}$), wie z. B. die Na- oder Ka-Salze der Oel- oder Stearinsäure, oder von natürlichen Fettsäuregemischen, die z. B. aus Kokosnuss- oder Talgöl gewonnen werden können. Ferner

sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z. B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyl-di-(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood, New Jersey, 1979 ; Dr. Helmut Stache « Tensid Taschenbuch », Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für Wirkstoffe der Formel I resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykol (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | – |
| Kaolin | – | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

a) Herstellung von 3,5-Dichlor-4-(3'-chlor-5'-trifluormethylpyridyl-2'-oxy)-N-(tetrahydropyranyl-2")-anilin

Ein Gemisch aus 17,8 g 3,5-Dichlor-4-(2'-chlor-5'-trifluormethylpyridyl-2'-oxy)-anilin und 5,3 g 5-Hydroxypentanal wird während 2 Stunden bei 85 °C gerührt. Nach dem Abkühlen wird das erhaltene Rohprodukt in Chloroform aufgenommen und die sich ergebende Lösung über Natriumsulfat getrocknet. Nach dem Eindampfen wird der Rückstand in Hexan aufgenommen. Aus dieser Lösung kristallisiert die Titelverbindung der Formel

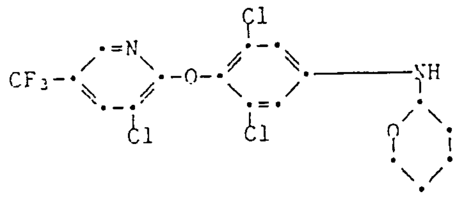

als weisse Kristalle mit einem Schmelzpunkt von 74-76 °C aus.

b) Herstellung von N-[3,5-Dichlor-4-(3'-chlor-5'-trifluormethylpyridyl-2'-oxy)-phenyl]-N-(tetrahydropyranyl-2")-N'-2-chlorbenzoylharnstoff

1,9 g 2-Chlorbenzoylisocyanat gelöst in 5 ml Diäthyläther werden zu einer Lösung von 4,4 g des vorstehend gemäss a) hergestellten Anilins in 40 ml Diäthyläther hinzugetropft. Nach zweistündigem Rühren des Ansatzes bei Raumtemperatur wird das auskristallisierte Reaktionsprodukt abfiltriert und mit wenig Diäthyläther gewaschen. Man erhält die Titelverbindung der Formel

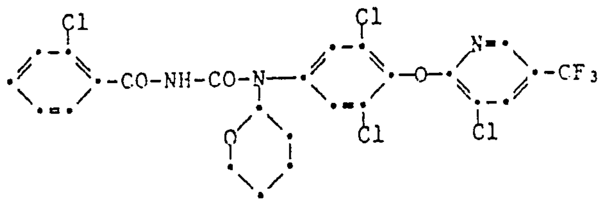

mit einem Schmelzpunkt von 152-153 °C (Verbindung Nr. 1)

Analog den vorstehend beschriebenen Arbeitsweisen werden die folgenden Verbindungen der Formel I hergestellt :

| Verbindung Nr. | | Smp. [°C] |
|---|---|---|
| 2 | 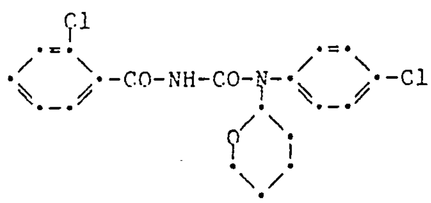 | 159-160 |
| 3 | 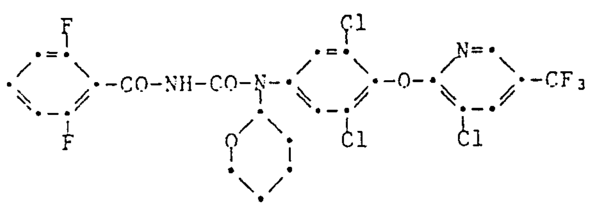 | 112-114 |
| 4 | 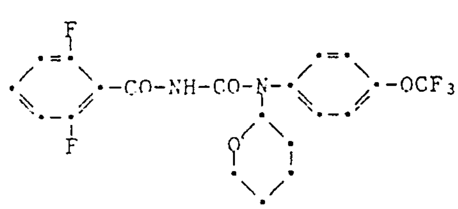 | 151-152 |

(Fortsetzung)

| Verbindung Nr. | | Smp. [°C] |
|---|---|---|
| 5 | 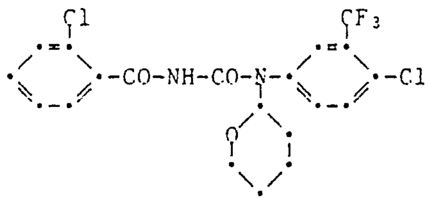 | 159-160 |
| 6 | 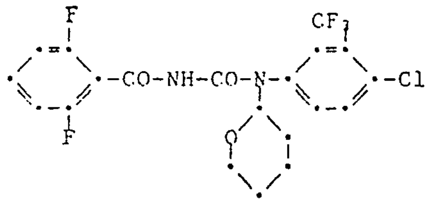 | 160-162 |
| 7 | 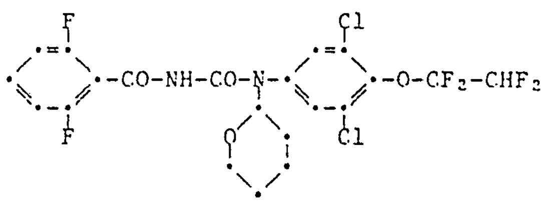 | 159-160 |
| 8 | 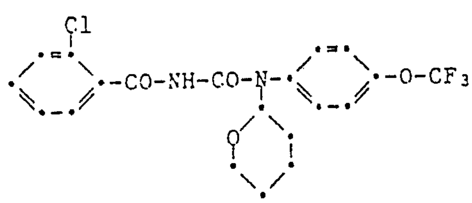 | 152-154 |
| 9 | 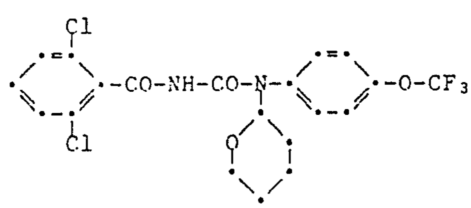 | 171-172 |
| 10 | 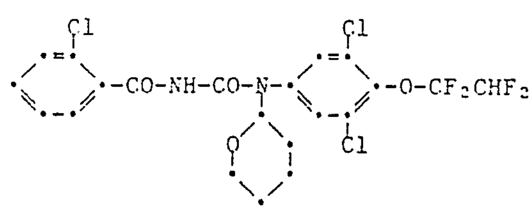 | 150-151 |
| 11 | 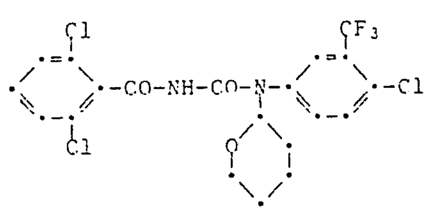 | 167-169 |

(Fortsetzung)

| Verbindung Nr. | | Smp. [°C] |
|---|---|---|
| 12 | 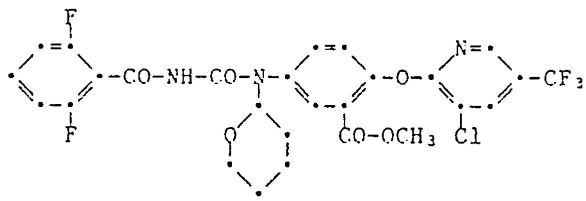 | 147-149 |
| 13 | 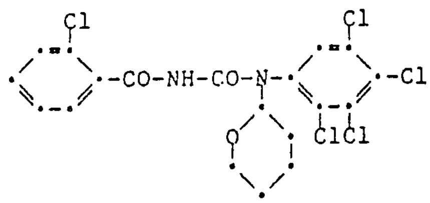 | 179-180 |
| 14 | 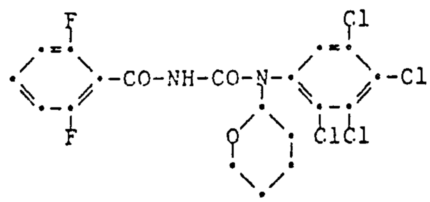 | 193-194 |
| 15 | 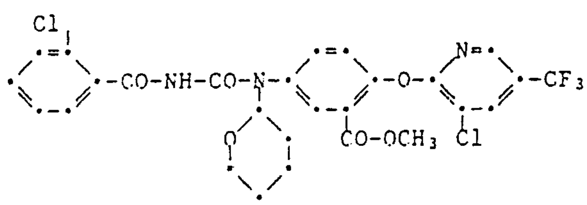 | 140-141 |

In entsprechender Weise wie vorstehend beschrieben sind auch die folgenden Verbindungen der Formel I herstellbar :

| Verbindung Nr. | |
|---|---|
| 16 | 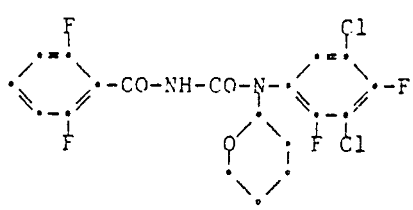 |
| 17 | 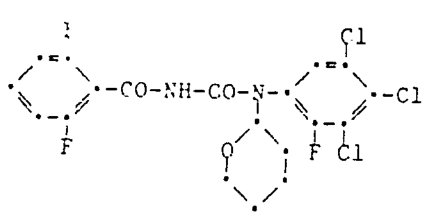 |

(Fortsetzung)

Verbindung Nr. | Smp. [°C]

18
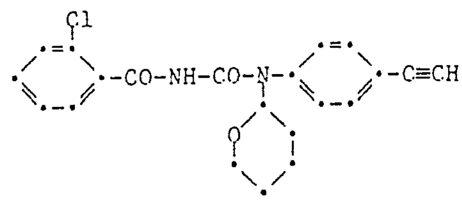

19
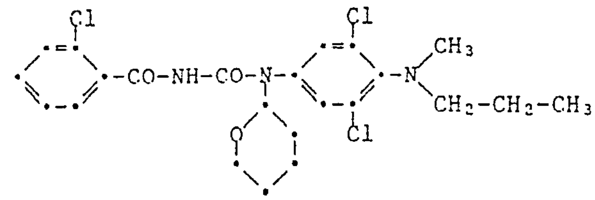

20
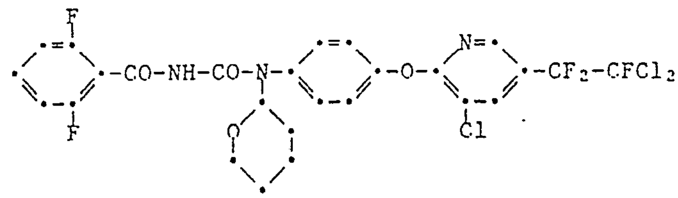

21
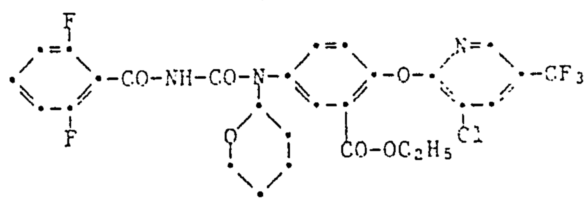

Beispiel 2 : Wirkung gegen Musca domestica

Je 50 g frisch zubereitetes Nährsubstrat für Maden werden in Becher eingewogen. Von einer 1 Gew.%igen acetonischen Lösung des betreffen den Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 3 : Wirkung gegen Lucilia sericata

Zu 9 ml eines Zuchtmediums wird bei 50 °C 1 ml einer die Aktivsubstanz enthaltenden wässrigen Zubereitung hinzugefügt. Nun werden ca. 30 frisch geschlüpfte Lucilia-sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata. So haben die vorstehenden Verbindungen Nr. 4 und 12 bei einer Konzentration von 50 ppm eine 100 %-ige Wirkung gegen Lucilia sericata.

Beispiel 4 : Wirkung gegen Aëdes aegypti

Auf die Oberfläche von 150 ml Wasser, das sich in einem Behälter befindet, wird so viel einer

0,1 Gew.%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 5 : Insektizide Frassgift-Wirkung

Ca 25 cm hohe eingetopfte Baumwollpflanzen werden mit wässrigen Wirkstoffemulsionen besprüht, die den Wirkstoff in Konzentrationen von 3 bis 200 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24 °C und 60 % relativer Luftfeuchtigkeit durchgeführt. Nach 120 Stunden wird die %-Mortalität der Test-Insekten bestimmt.

Beispiel 6 : Wirkung gegen Epilachna varivestis

Etwa 15-20 cm hohe Phaseolus vulgaris-Pflanzen (Buschbohnen) werden mit wässrigen, den zu prüfenden Wirkstoff in Konzentrationen von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28 °C und 60 % relativer Luftfeuchtigkeit durchgeführt.

Nach 2 und 3 Tagen wird die % Mortalität bestimmt. Zur Auswertung hinsichtlich allfälligem Frass-Schaden (Antifeeding-Effekt), Entwicklungs- und Häutungsstörungen werden die Versuchstiere während weitere 3 Tage beobachtet.

Verbindungen der Formel I gemäss Beispiel 1 zeigen gute Wirkung im obigen Test.

Beispiel 7 : Ovizide Wirkung auf Heliothis virescens

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 800 ppm ergibt.

In diese wirkstoffhaltige Emulsion werden eintägige Eigelege von Heliothis auf Cellophan während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt. Zur Auswertung wird die zur 100 %igen Abtötung der Eier erforderliche minimale Wirkstoffkonzentration bestimmt.

Verbindungen der Formel I gemäss Beispiel 1 zeigen gute Wirkung in obigem Test.

Beispiel 8 : Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28 °C belassen. Nach 6 Tagen wird der prozentuale Schlupf aus den behandelten Eiern bewertet und die %-Mortalität bestimmt.

Verbindungen der Formel I gemäss Beispiel 1 zeigen gute Wirkung in obigem Test.

Beispiel 9 : Reproduktions-Beeinflussung von Anthonomus grandis

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in acetonische Lösungen, enthaltend 12,5 bis 200 ppm des zu prüfenden Wirkstoffes, eingetaucht. Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die

Bonitierung erfolgt anhand der Verminderung der Anzahl abgelegter Eier und der daraus geschlüpften Larven im Vergleich zu unbehandelten Kontrollen.

Beispiel 10 : Akarizide Wirkung

Phaseolus vulgaris Pflanzen werden 12 Stunden vor dem Test auf akarizide Wirkung mit einem infestierten Blattstück aus einer Massenzucht von Tetranychus urticae belegt. Die übergerlaufenen beweglichen Stadien werden aus einem Chromatographiezerstäuber mit den emulgierten Testpräparaten derart besprüht, dass kein Ablaufen der Spritzbrühe eintritt. Die jeweils verwendeten Emulsionszubereitungen weisen eine Wirkstoffkonzentration von 800 ppm auf. Nach zwei und 7 Tagen werden Larven, Adulte und Eier unter dem Binokular auf lebende und tote Individuen ausgewertet ; das Ergebnis wird in Prozenten ausgedrückt.

Während der « Haltezeit » stehen die behandelten Pflanzen in Gewächshauskabinen bei 25 °C.

Verbindungen der Formel I gemäss Beispiel 1 zeigen im obigen Test gute Wirkung.

Beispiel 11 : Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung, enthaltend 12,5 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25 °C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Biologische Ergebnisse

Die nachstehende Tabelle zeigt die Ergebnisse biologischer Prüfungen erfindungsgemässer Verbindungen auf der Basis der obigen biologischen Beispiele 5 und 9. Die Auswertung der Versuche erfolgt anhand der erhaltenen %-Mortalität unter Verwendung von folgendem Bewertungs-Index :

A : 80-100 % Mortalität bei einer Konzentration von 3 ppm der geprüften Verbindung.
B : 80-100 % Mortalität bei einer Konzentration von 12,5 ppm der geprüften Verbindung.
C : 80-100 % Mortalität bei einer Konzentration von 50 ppm der geprüften Verbindung.
D : 80-100 % Mortalität bei einer Konzentration von 100 ppm der geprüften Verbindung.
E : 80-100 % Mortalität bei einer Konzentration von 200 ppm der geprüften Verbindung.

| Verbindung Nr. | pestizide Wirksamkeit | | |
|---|---|---|---|
| | Spodoptera (Beispiel 5) | Heliothis (Beispiel 5) | Anthonomus (Beispiel 9) |
| 1 | B | C | B |
| 3 | A | B | B |
| 4 | - | - | B |
| 5 | E | - | - |
| 6 | B | - | C |
| 7 | B | - | - |
| 8 | C | C | B |
| 10 | C | E | D |
| 12 | A | B | B |
| 13 | - | - | E |
| 15 | A | B | C |

## Patentansprüche

1. Verbindung der Formel I

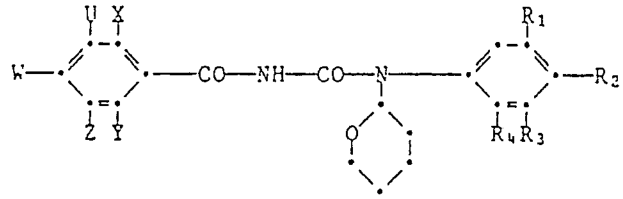

(I)

worin

$R_1$ Wasserstoff, Halogen, $C_1$-$C_6$-Alkyl, $C_1$-$C_3$-Halogenalkyl mit 1 bis 7 Halogenatomen, $C_1$-$C_6$-Alkoxy, C.-$C_6$-Alkylthio, $C_1$-$C_3$-Halogenalkoxy mit 1 bis 7 Halogenatomen, Nitro, Cyano, Aethinyl oder eine der Gruppen

$-CO-OR_5$ oder 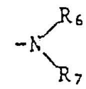

wobei $R_5$, $R_6$ und $R_7$ für $C_1$-$C_6$-Alkyl steht ;

$R_2$, $R_3$ $R_4$ unabhängig voneinander Wasserstoff, Halogen, $C_1$-$C_6$-Alkyl, $C_1$-$C_3$-Halogenalkyl mit 1 bis 7 Halogenatomen, $C_1$-$C_6$-Alkoxy, $C_1$-$C_6$-Alkylthio, $C_1$-$C_3$-Halogenalkoxy mit 1 bis 7 Halogenatomen, Nitro, Cyano, Aethinyl oder eine der Gruppen

$-CO-OR_5$ oder 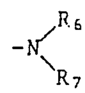

wobei $R_5$, $R_6$ und $R_7$ für $C_1$-$C_6$-Alkyl steht,

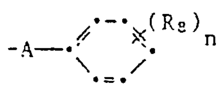

wobei —A— für —O— oder —S—, $R_8$ für Halogen, Methyl, Nitro, $CF_3$ oder Cyano und n für eine Zahl 0, 1, 1 oder 3 steht, oder

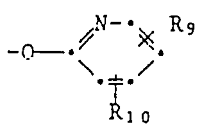

wobei $R_9$ und $R_{10}$ unabhängig voneinander für Wasserstoff, Chlor, Trifluormethyl oder einen mit Fluor- und/oder Chlor perhalogenierten Aethylrest stehen , und

U, X, Y, Z und W unabhängig voneinander Wasserstoff, Halogen, $C_1$-$C_3$-Alkyl, $C_1$-$C_3$-Alkoxy oder C.-$C_3$-Alkylthio

bedeuten.

2. Verbindung der Formel I gemäss Anspruch 1, dadurch gekennzeichnet, dass

R. Wasserstoff, Fluor, Chlor, Trifluormethyl, —CO—$OCH_3$ oder —CO—$OC_2H_5$ ;

$R_2$ Wasserstoff, Fluor, Chlor, $C_1$-$C_2$-Halogenalkyl mit 1 bis 5 Chlor- und/oder Fluoratomen, C.-C.-Halogenalkoxy mit 1 bis 5 Chlor- und/oder Fluoratomen, Cyano, Aethinyl, oder eine der Gruppen

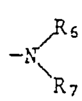

wobei $R_6$ und $R_7$ für C.-$C_3$-Alkyl steht,

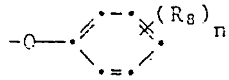

wobei $R_8$ für Fluor oder Chlor und n für n eine Zahl 0, 1 oder 2 steht,

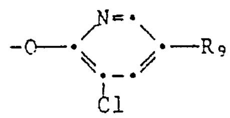

wobei $R_9$ für Wasserstoff, Chlor, Trifluormethyl, $—CF_2—CF_2Cl$ oder $—CF_2—CFCl_2$ steht,

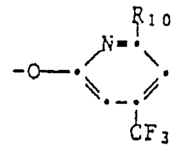

wobei $R_{10}$ für Wasserstoff oder Chlor steht ;

$R_3$ und $R_4$ unabhänging voneinander Wasserstoff, Halogen, Trifluormethyl, Methyl oder Aethyl ;

U, Z und W Wasserstoff ;

X Fluor, Chlor, Brom oder $C_1$-$C_3$-Alkyl ; und

Y Wasserstoff, Fluor, Chlor, Brom oder $C_1$-$C_3$-Alkyl

bedeuten.

3. Verbindung der Formel I gemäss Anspruch 2, dadurch gekennzeichnet, dass

$R_1$ Wasserstoff, Fluor, Chlor oder $—CO—OCH_3$ ;

$R_2$ Wasserstoff, Fluor, Chlor, Trifluormethyl, Aethinyl, Trifluormethoxy, $—O—CF_2—CF_2Cl$, $—O—CF_2—CHF_2$, $—O—CF_2—CFCl_2$ oder eine der Gruppen

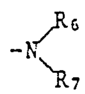

wobei $R_6$ für Methyl und $R_7$ für $C_1$-$C_3$-Alkyl steht,

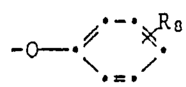

wobei $R_8$ für Wasserstoff, Fluor oder Chlor steht,

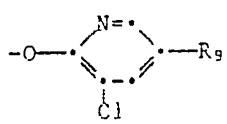

wobei $R_9$ für Trifluormethyl oder $—CF_2—CFCl_2$ steht, oder

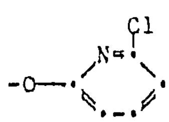

$R_3$ und $R_4$ unabhängig voneinander Wasserstoff, Fluor, Chlor, Trifluormethyl oder Methyl ;

U, Z und W Wasserstoff ;

X Fluor oder Chlor ; und

Y Wasserstoff, Fluor oder Chlor

bedeuten.

4. Verbindung der Formel I gemäss Anspruch 3, dadurch gekennzeichnet, dass

R- Chlor oder Trifluormethyl ;

$R_2$ Chlor, Trifluormethyl, Trifluormethoxy, $—O—CF_2—CHF_2$ oder die Gruppe

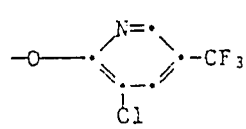

$R_3$ Wasserstoff, Chlor oder $—CO—OCH_3$ ;

$R_4$ Wasserstoff ;

U, Z und W Wasserstoff ;
X Fluor oder Chlor ; und
Y Wasserstoff oder Fluor
bedeuten.

5. Verbindung gemäss Anspruch 4 der Formel

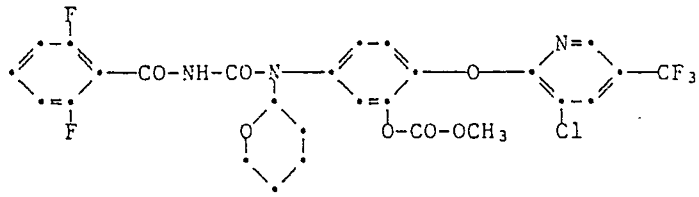

6. Verbindung gemäss Anspruch 4 der Formel

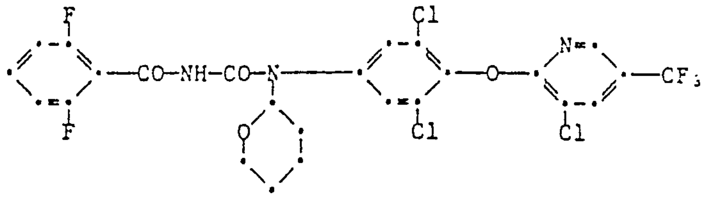

7. Verbindung gemäss Anspruch 4 der Formel

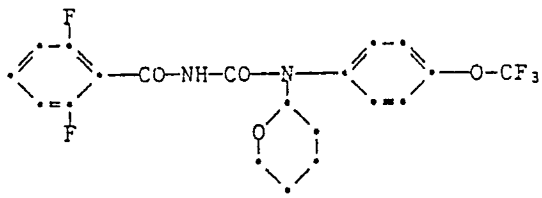

8. Verfahren zur Herstellung einer Verbindung gemäss den Ansprüchen 1 bis 7, dadurch gekennzeichnet, dass man eine Verbindung der Formel II

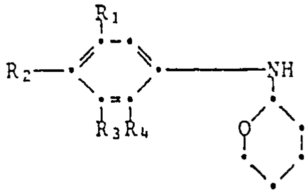

(II)

mit einer Verbindung der Formel III

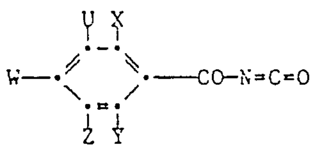

(III)

umsetzt, wobei in den Formeln II und III die Reste $R_1$, $R_2$, $R_3$, $R_4$, U, X, Y, Z und W die in den Ansprüchen 1 bis 4 angegebenen Bedeutungen haben.

9. Schädlingsbekämpfungsmittel, welches als aktive Komponente eine Verbindung gemäss den Ansprüchen 1 bis 7 zusammen mit geeigneten Trägern und/oder anderen Zuschlagstoffen enthält.

10. Verwendung einer Verbindung gemäss den Ansprüchen 1 bis 7 zur Bekämpfung von Schädlingen, vorzugsweise von pflanzenschädigenden Insekten und Vertretern der Ordnung Akarina, unter Vorbehalt von Art. 52 (4) EPÜ.

11. Verfahren zur Bekämpfung von Insekten und Vertretern der Ordnung Akarina, dadurch gekennzeichnet, dass man die Schädlinge und/oder deren Aufenthaltsort mit einer pestizid wirksamen Menge einer Verbindung der Formel I gemäss den Ansprüchen 1 bis 7 oder eines diese enthaltenden Mittels behandelt, unter Vorbehalt von Art. 52(4) EPÜ.

## Claims

1. A compound of formula I

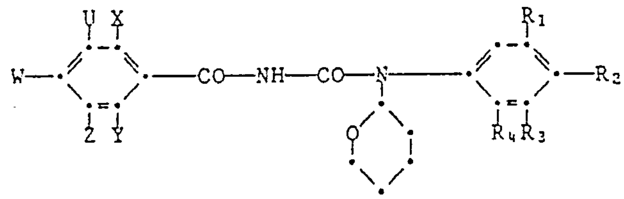

(I)

wherein

$R_1$ is hydrogen, halogen, $C_1$-$C_6$-alkyl, $C_1$-$C_3$haloalkyl containing 1 to 7 halogen atoms, $C_1$-$C_6$alkoxy, $C_1$-$C_6$alkylthio, $C_1$-$C_3$halogalkoxy containing 1 to 7 halogen atoms, nitro, cyano, ethynyl or one of the groups

$-CO-OR_5$ or 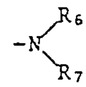

wherein $R_5$, $R_6$ and $R_7$ are $C_1$-$C_6$alkyl ;

$R_2$, $R_3$ and $R_4$ are each independently hydrogen, halogen, $C_1$-$C_6$alkyl, $C_1$-$C_3$haloalkyl containing 1 to 7 halogen atoms, $C_1$-$C_6$alkoxy, $C_1$-$C_6$alkylthio, $C_1$-$C_3$haloalkoxy containing 1 to 7 halogen atoms, nitro, cyano, ethynyl or one of the groups

$-CO-OR_5$ or 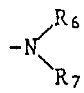

wherein $R_5$, $R_6$ and $R_7$ are $C_1$-$C_6$alkyl,

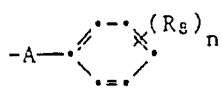

wherein —A— is —O— or —S—, $R_8$ is halogen, methyl, nitro, $CF_3$ or cyano and n is 0, 1, 2 or 3, or

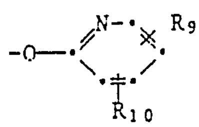

wherein each of $R_9$ and $R_{10}$ independently is hydrogen, chlorine, trifluoromethyl or an ethyl radical perhalogenated with fluorine and/or chlorine ; and

U, X, Y, Z and W are each independently hydrogen, halogen, $C_1$-$C_3$alkyl, $C_1$-$C_3$alkoxy or $C_1$-$C_3$alkylthio.

2. A compound of formula I according to claim 1, wherein

$R_1$ is hydrogen, fluorine, chlorine, trifluoromethyl, $-CO-OCH_3$ or $-CO-OC_2H_5$ ;

$R_2$ is hydrogen, fluorine, chlorine, $C_1$-$C_2$haloalkyl containing 1 to 5 chlorine and/or fluorine atoms, $C_1$-$C_2$haloalkoxy containing 1 to 5 chlorine and/or fluorine atoms, cyano, ethynyl or one of the groups

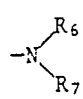

wherein $R_6$ and $R_7$ are $C_1$-$C_3$alkyl,

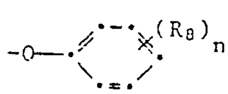

wherein $R_8$ is fluorine or chlorine and n is 0,1 or 2,

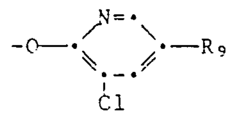

wherein $R_9$ is hydrogen, chlorine, trifluoromethyl, $-CF_2-CF_2Cl$ or $-CF_2-CFCl_2$,

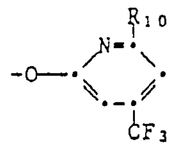

wherein $R_{10}$ is hydrogen or chlorine ;
$R_3$ and $R_4$ are each independently hydrogen, halogen, trifluoromethyl, methyl or ethyl ;
U, Z and W are hydrogen ;
X is fluorine, chlorine, bromine or $C_1$-$C_3$alkyl ; and
Y is hydrogen, fluorine, chlorine, bromine or $C_1$-$C_3$alkyl.

3. A compound of formula I according to claim 2, wherein
$R_1$ is hydrogen, fluorine, chlorine or $-CO-OCH_3$ ;
$R_2$ is hydrogen, fluorine, chlorine, trifluoromethyl, ethynyl, trifluoromethoxy, $-O-CF_2-CF_2Cl$, $-O-CF_2-CHF_2$, $-O-CF_2-CFCl_2$ or one of the groups

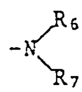

wherein $R_6$ is methyl and $R_7$ is $C_1$-$C_3$alkyl,

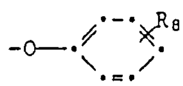

wherein $R_8$ is hydrogen, fluorine or chlorine,

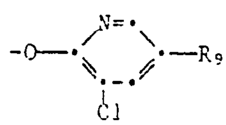

wherein $R_9$ is trifluoromethyl or $-CF_2-CFCl_2$, or

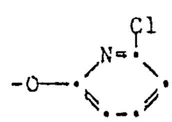

$R_3$ and $R_4$ are each independently hydrogen, fluorine, chlorine, trifluoromethyl or methyl ;
U, Z and W are hydrogen ;
X is fluorine or chlorine ; and
Y is hydrogen, fluorine or chlorine.

4. A compound of formula I according to claim 3, wherein
$R_1$ is chlorine or trifluoromethyl ;
$R_2$ is chlorine, trifluoromethyl, trifluoromethoxy, $-O-CF_2-CHF_2$ or the group

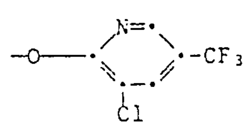

$R_3$ is hydrogen, chlorine or $-CO-OCH_3$ ;
$R_4$ is hydrogen ;
U, Z and W are hydrogen ;
X is fluorine or chlorine ; and

Y is hydrogen or fluorine.

5. A compound according to claim 4 of the formula

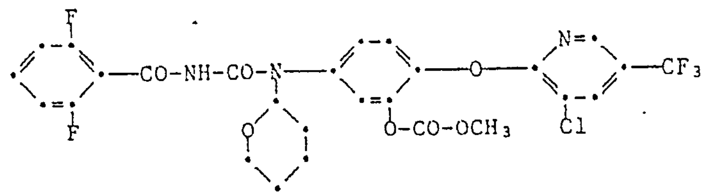

6. A compound according to claim 4 of the formula

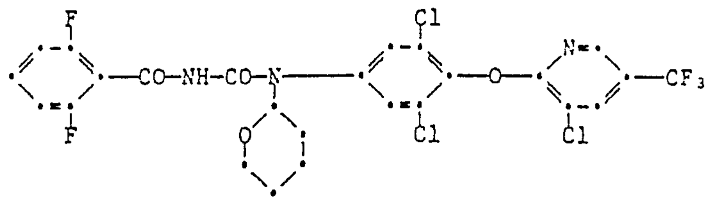

7. A compound according to claim 4 of the formula

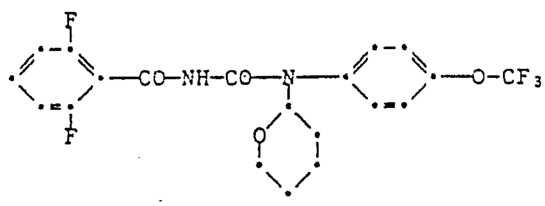

8. A process for the preparation of a compound according to claims 1 to 7, which process comprises reacting a compound of formula II

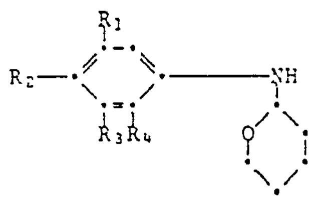

(II)

with a compound of formula III

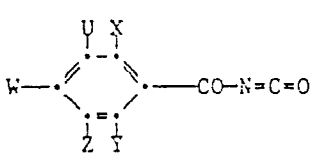

(III)

in which formulae II and III the radicals $R_1$, $R_2$, $R_3$, $R_4$, U, X, Y, Z and W are as defined in claims 1 to 4.

9. A pesticidal composition which contains, as active ingredient, a compound according to claims 1 ot 7, together with suitable carriers and/or other adjuvants.

10. Use of a compound according to claims 1 to 7 for controlling pests, preferably plant-destructive insects and representatives of the order Acarina, subject to the provisions of Art. 52(4) EPC.

11. A method of controlling insects and representatives of the order Acarina, which method comprises treating the pests and/or the locus thereof with a pesticidally effective amount of a compound of formula I according to claims 1 to 7 or of a composition containing such a compound, subject to the provisions of Art. 52(4) EPC.

**Revendications**

1. Composé de formule I

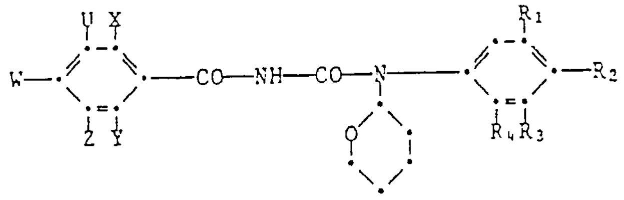 (I)

où

$R_1$ représente un hydrogène, halogène, alcoyle en $C_1$ à $C_6$, halogènalcoyle en $C_1$ à $C_3$ avec de 1 à 7 atomes d'halogène, alcoxy en $C_1$ à $C_6$, alcoylthio en $C_1$ à $C_6$, halogènalcoxy en $C_1$ à $C_3$ avec de 1 à 7 atomes d'halogène, nitro, cyano, éthynyle ou l'un des groupes

$-CO-OR_5$ ou 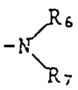

où $R_5$, $R_6$ et $R_7$ représentent un alcoyle en $C_1$ à $C_6$ ;

$R_2$, $R_3$ et $R_4$ représentent indépendamment l'un de l'autre un hydrogène, halogène, alcoyle en $C_1$ à $C_6$, halogènalcoyle en $C_1$ à $C_3$ avec de 1 à 7 atomes d'halogène, alcoxy en $C_1$ à $C_6$, alcoylthio en $C_1$ à $C_6$, halogènalcoxy en $C_1$ à $C_3$ avec de 1 à 7 atomes d'halogène, nitro, cyano, éthynyle ou l'un des groupes

$-CO-OR_5$ ou 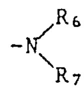

où $R_5$, $R_6$ et $R_7$ représentent un alcoyle en $C_1$ à $C_6$,

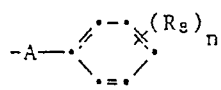

où —A— représente —O— ou —S—, $R_8$ représente un halogène, méthyle, nitro, $CF_3$ ou cyano et n représente le nombre 0, 1, 2 ou 3, ou

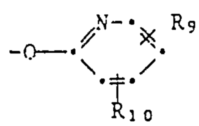

où $R_9$ et $R_{10}$ représentent indépendamment l'un de l'autre un hydrogène, chlore, trifluorométhyle ou un radical éthyle perhalogéné avec du fluor et/ou du chlore ; et

U, X, Y, Z et W représentent indépendamment l'un de l'autre un halogène, alcoyle en $C_1$ à $C_3$, alcoxy en $C_1$ à $C_3$ ou alcoylthio en $C_1$ à $C_3$.

2. Composé de formule I selon la revendication 1, caractérisé en ce que

$R_1$ représente un hydrogène, fluor, chlore, trifluorométhyle, $—CO—OCH_3$ ou $—CO—OC_2H_5$ ;

$R_2$ représente un hydrogène, fluor, chlore, halogènalcoyle en $C_1$ et $C_2$ avec de 1 à 5 atomes de chlore, et/ou de fluor, halogènealcoxy en $C_1$ à $C_2$ avec de 1 à 5 atomes de chlore et/ou de fluor, cyano, éthynyle, ou l'un des groupes

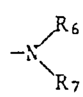

où $R_6$ et $R_7$ représentent un alcoyle en $C_1$ à $C_3$,

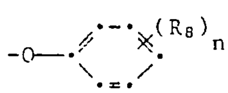

où $R_8$ représente un fluor ou un chlore et n représente le nombre 0, 1 ou 2,

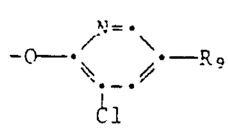

où $R_9$ représente un hydrogène, chlore, trifluorométhyle, $—CF_2—CF_2Cl$ ou $—CF_2—CFCl_2$,

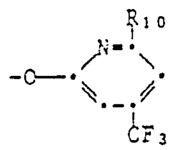

où $R_{10}$ représente un hydrogène ou un chlore ;

$R_3$ et $R_4$ représentent indépendamment l'un de l'autre un hydrogène, halogène, trifluorométhyle, méthyle ou éthyle ;

U, Z et W représentent un hydrogène ;

X représente un fluor, chlore, brome ou alcoyle en $C_1$ à $C_3$ ;

Y représente un hydrogène, fluor, chlore, brome ou alcoyle en $C_1$ à $C_3$.

3. Composé de formule I selon la revendication 2, caractérisé en ce que

$R_1$ représente un hydrogène, fluor, chlore ou $—CO—OCH_3$ ;

$R_2$ représente un hydrogène, fluor, chlore, trifluorométhyle, éthynyle, trifluorométhoxy, $—O—CF_2—CF_2Cl$, $—O—CF_2—CHF_2$, $—O—CF_2—OFCl_2$ ou l'un des groupes

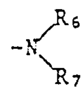

où $R_6$ représente un méthyle et $R_7$ un alcoyle en $C_1$ à $C_3$,

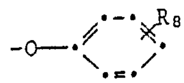

où $R_8$ représente un hydrogène, fluor ou chlore,

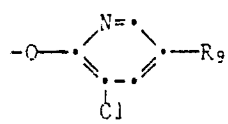

où $R_9$ représente un trifluorométhyle ou $—CF_2—CFCl_2$, ou

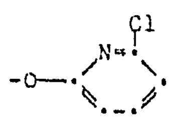

$R_3$ et $R_4$ représentent indépendamment l'un de l'autre un hydrogène, fluor, chlore, trifluorométhyle ou méthyle ;

U, Z et W représentent un hydrogène ;

X représente un fluor ou un chlore ; et

Y représente un hydrogène, fluor ou chlore.

4. Composé de formule I selon la revendication 3, caractérisé en ce que

$R_1$ représente un chlore ou un trifluorométhyle ;

$R_2$ représente un chlore, trifluorométhyle, trifluorométhoxy, $—O—CF_2—CHF_2$ ou le groupe

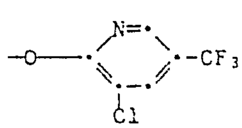

$R_3$ représente un hydrogène, chlore ou $—CO—OCH_3$ ;

$R_4$ représente un hydrogène ;

U, Z et W représentent un hydrogène ;

X représente un fluor ou un chlore ; et

Y représente un hydrogène ou un fluor.

5. Composé selon la revendication 4, de formule

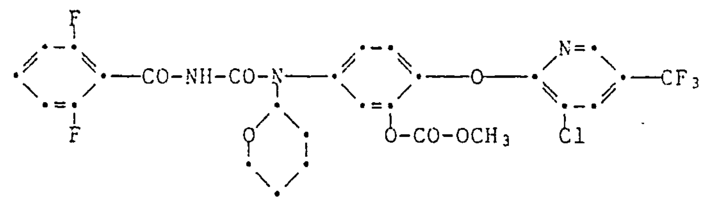

6. Composé selon la revendication 4 de formule

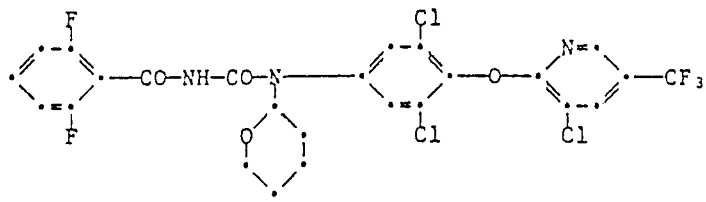

7. Composé selon la revendication 4, de formule

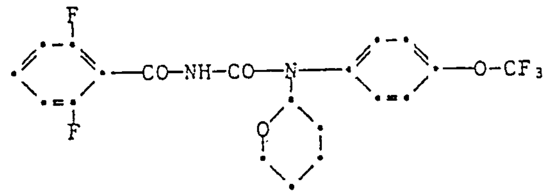

8. Procédé de préparation d'un composé selon les revendications 1 à 7, caractérisé en ce qu'on fait réagir un composé de formule II

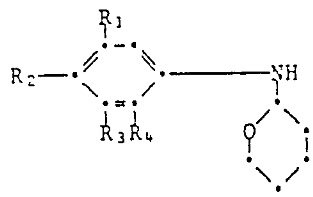

(II)

avec un composé de formule III

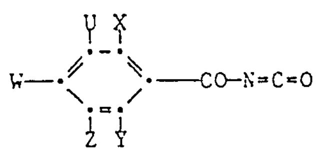

(III)

où dans les formules II et III les radicaux $R_1$, $R_2$, $R_3$, $R_4$, U, X, Y, Z et W ont les significations données dans les revendications 1 à 4.

9. Agent de lutte antiparasitaire qui contient comme composant actif un composé selon les revendications 1 à 7 avec des supports et/ou autres additifs appropriés.

10. Application d'un composé selon les revendications 1 à 7 à la lutte contre les parasites, de préférence les insectes qui causent des dommages aux plantes et les représentants de l'ordre des Acariens, en tenant compte de l'article 52(4) EPU.

11. Procédé de lutte contre les insectes et les représentants de l'ordre des Acariens, caractérisé en ce qu'on traite les parasites et/ou leurs lieux d'habitat avec une quantité active comme pesticide d'un composé de formule I selon les revendications 1 à 7 ou d'un agent qui les contient en tenant compte de l'article 52(4) EPU.